# EUROPEAN PATENT SPECIFICATION

(11) **EP 1 842 484 B1**
(45) Date of publication and mention of the grant of the patent: **11.11.2009**
(21) Application number: 07105552.9
(22) Date of filing: 03.04.2007
(51) Int. Cl.: A61B 5/0205, A61B 17/16, A61C 1/00, H02P 6/08, H02P 25/02

(54) **Control system of an electric motor for surgical applications**
Steuersystem eines Elektromotors für chirurgische Anwendungen
Système de commande d'un moteur électrique pour des applications chirurgicales

(30) Priority: 04.04.2006 IT MI20060652
(43) Date of publication of application: 10.10.2007
(73) Proprietor: I.D.I. Evolution S.r.l., 20052 Monza MI (IT)
(72) Inventor: PIANTONI, Angiolino, 20050, LESMO (Milan) (IT)
(74) Representative: Martegani, Franco

(56) References cited:
- WO-A-2005/055824
- US-A- 4 995 877
- US-A- 5 689 159

## Description

The present invention relates to a control system of an electric motor for surgical applications.

In particular, the present invention relates to a control system of an electric motor for surgical applications in the dental field for the insertion and fitting of dental implants in implant treatment, with special functions supported by scientific works.

Surgical motors already exist in the known art, produced from brushless and induction motors suitably driven by electronic control cards.

US 5, 689, 159 discloses a control system of an electric motor according to the preamble of claim 1.

An objective of the present invention is to provide a control system for a surgical motor in which the motor can be programmed in relation to the type of pre-established intervention which can depend on the anamnestic data of the patient or pre-operating data revealed radiologically; information on the intervention to be effected can be set up, so that the motor operates under certain conditions depending on the conditions established. Furthermore, the electronics allows useful information to be obtained for programming the motor, referring to the rotation rate and quantity of absorbed current which when correctly parameterized suggests the type of substrate of interest and resistance of the instrument or device used, and also the interface with the instrument kit suggests the correct sequence of the instruments to be used. At the same time the electronics indicates the correct type of tool (cutter) to be used for the intervention. The data acquired by the software are constantly monitored and visualized on a monitor in the form of numbers (values expressed in Newton) and graphs indicating the behaviour of the instrument as it proceeds in the substrate of interest. The values controlled are the number of revs, the number of revs per minute and the electric absorption for keeping the previous value constant (expression of the resistance encountered by the instrument). Furthermore, a series of functioning values of the motor detected, such as for example the rotation rate, current absorbed, position of the rotation of the motor, can determine the functioning conditions of the system and therefore determine characteristics of the implant to be effected.

These and other objectives are achieved by the present invention by providing a control system of an electric motor for surgical applications, according to claim 1, to which reference should be made for the sake of brevity.

Further characteristics of the invention are defined in the other claims enclosed with the present invention.

Further objectives and advantages of the present invention will appear evident from the following description and enclosed drawing, provided for purely illustrative and non-limiting purposes, in which:
- figure 1 represents a block scheme of the power supply section of the control system according to the present invention;
- figure 2 represents a block scheme of the driving section of the motor of the control system according to the present invention;
- figure 3 represents a block scheme of the visualization section of the control system according to the present invention;
- figure 4 represents a block scheme of the driving section of a water flow pump of the control system according to the present invention.

With particular reference to the above figures, the system according to the present invention comprises a console, an electric micromotor, an intelligent tray and a pedal.

Furthermore, the console comprises an electronic power supply section or card 2, an electronic motor control section or card 3, an electronic logic and display section or card 4 and an electronic hydraulic drive section or card 5 for a peristaltic pump 6 for the cooling liquid.

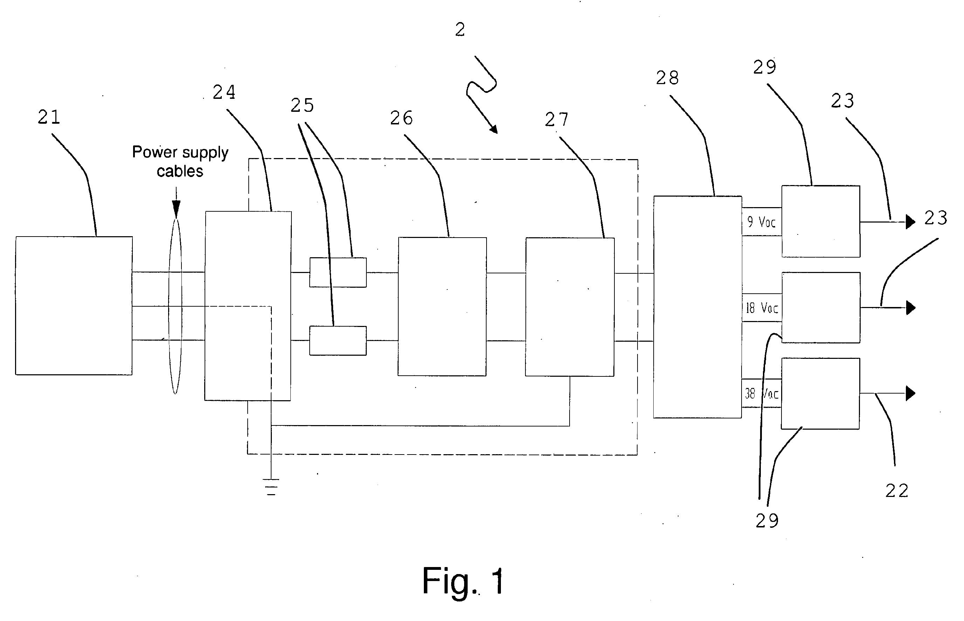

Figure 1 illustrates the power supply section of the control system which comprises a series of components starting from a power supply system 21 for example at 230V AC as far as the control output 22 of the direct current at about 40 V which charges the motor control and at the charge outputs 23 with a direct current at 5 V and 12 V which charge the logic display circuit. In particular, the circuit preferably comprises an input connector 24, a pair of fuses 25, a bipolar switch 26, a filter 27, a transformer phase 28 and a rectifier step 29 for each output.

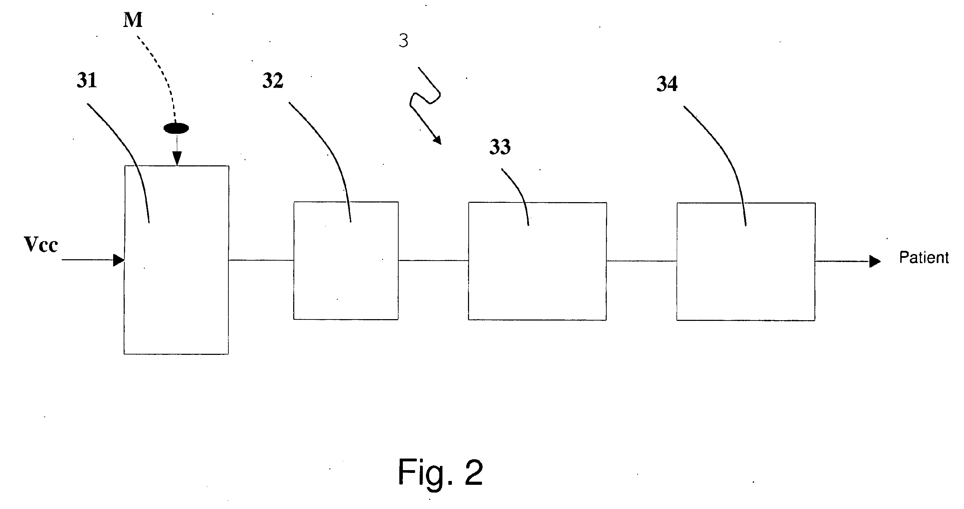

Figure 2 illustrates the motor control section which is capable of providing a power signal which adequately drives the Brushless motor. In particular, the section comprises a pilot circuit 31, a connector 32 of the pilot circuit to the motor 33 which activates a counterangle 34.

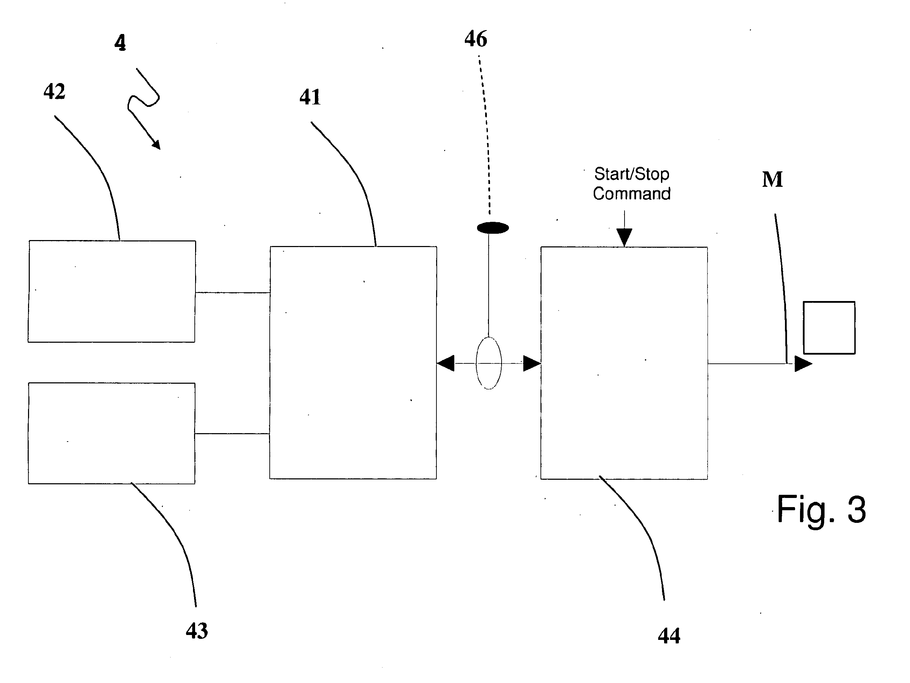

Figure 3 illustrates the logic and display section 4 which comprises a micro-controller 41 which controls a display 42 and which receives commands from a keyboard 43. Furthermore, the logic section comprises a control circuit of the motor 44, which is associated with the drive circuit of the motor of the control section, and which receives a start/stop command for the activation of the motor.

The start is provided by the motor control section, communicated to the logic section which determines the parameters established by means of a serial communication of the master-slave type wherein the master is the motor control section and the slave is the logic section. The power supplied is regulated by means of said micro-controller.

The logic section visualizes and controls the whole functioning of the device, in particular, once activated, the pedal 46 supplies the consent and parameters established for the machine and manages the functions described.

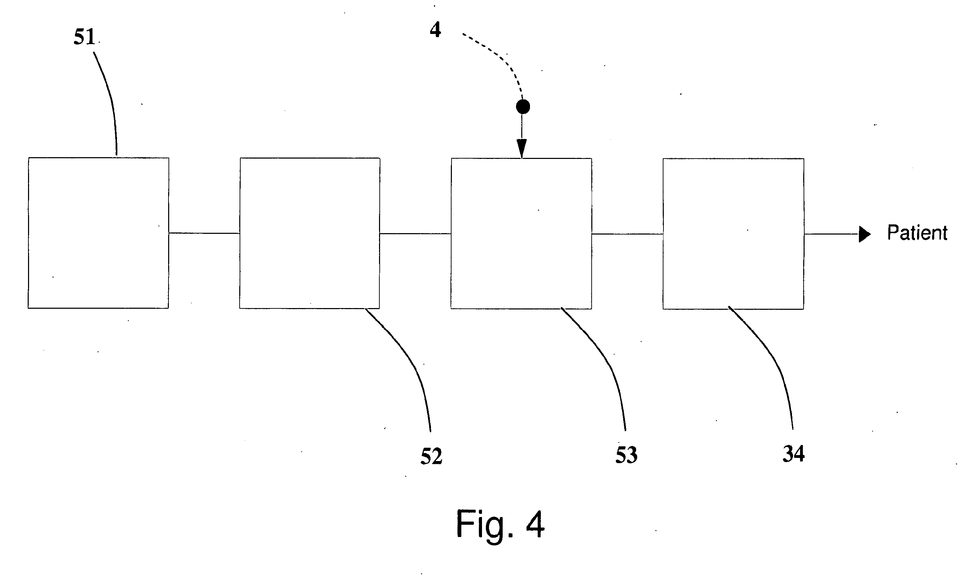

Figure 4 illustrates the drive section 5 of a water flow pump of the control system according to the present invention.

The pump section allows the water flow which starts from a bag 51 containing a physiological solution, to reach the counterangle 34 in a controlled manner by means of a tube 52 and a pump 53 and exerts its functions, i.e. washing of the intervention area, cooling of the intervention area and cooling of the cutters. To allow the water flow to efficiently exert its functions, the connection tube between the bag and the pump must be correctly assembled.

The console physically consists of four elements, a front panel, a rear panel, a U-folded base and a U-folded top, all advantageously made of stainless steel plate. The power supply and control electronics are preferably assembled on the base and a manostat which is used for the start command, a connector used for the optional multifunctional pedal, the peristaltic pump, the filter 27-fuse-holder 25 connector for the connection of the power supply system, the charge switch, are housed on the rear panel. The front panel advantageously supports the logic section.

Following pressure on the pedal, after selecting the operating function with the parameters established on the keyboard, the motor must rotate at the rate and with the torque set, the peristaltic pump must contemporaneously also be started and a jet of water is therefore released with the intensity preselected by the operator regulating the rate of the pump motor.

Data are transmitted from the motor which reach the tray and visualize the ideal path by means of the indicators (luminous) in relation to the pre-established program. Sensors (tactile or other) in the tray perceive the withdrawn instrument and verify its congruity. The preparation phases of the implant tunnel provide the operator and software with information on the conditions (referring to the type of bone identified) and, through indications communicated to the tray and through the display, can always suggest a different clinical approach.

The tray communicates with the console through a connection wire and every time the user withdraws a cutter it is indicated on the display whether the cutter to be used is the right one or not depending on the parameters selected. In relation to these, the type of cutter to be used with respect to the type of implant initially selected by the operator, is indicated in the display of the console.

The premises for the good functioning of a system of this type is a knowledge of the type of bone referring to "its density" and the correct clinical approach to be adopted in relation to:
1. operative choice (selection of the charge time)
2. patient's anamnesis (pathologies inherent to the bone tissue)
3. compression value to be applied to the device during its application.

The system according to the present invention is equipped with a specific reader/tapper capable of reading the type of bone, for example the hardness, and completely automatically guiding the operator in the various preparation phases of the implant site and fitting of the implant, establishing as parameters for example the length of the implant, the pitch and diameter. In this way, the system is capable of guiding the user in the selection of the operating phases in a completely automatic way.

In particular, the intervention sequences are organized as follows:
● selection of the type of implant;
● perforation with countersunk perforation;
● reading of the type of bone with a specific reader;
● tapping or re-perforation of the site until the final fitting phase of the implant.

During the fitting of the implant, the system visualizes data for knowing the type of compression of the implant, in relation to the type of bone and the type of primary stability of the implant, and when the type of implant to be fitted during the screwing phase is known, the system will stop when the depth established in the initial phase has been reached.

In all the operating phases, the system will indicate the type of instrument to be used and will verify if the user is using the right instrument by means of the surgical intelligent tray connected to the console and consequently to the logic card.

The surgical tray comprises a base plate which perceives by means of an electronic/electromechanical sensor of the cutters are present, and indicates the lack thereof by means of an acoustic and/or visual warning signal (for example a buzzer or LED) and provides the micro-controller with this information. By establishing the type of implant to be fitted and knowing the type of bone, the system will indicate the type of cutter to be used by means of acoustic/visual indications.

In each operating phase, the system indicates to the tray the type of cutter or tapper to be used in the various sequences, which indicates if the cutter removed is the right one and it always indicates the right cutter to be used. The tray preferably consists of a medical plastic container and an anodized aluminum plate suitable for containing the numerous components of the method.

The detection of the instrument is preferably effected by means of sensors/microswitches/optical devices/capacitive devices. With these premises, it is possible to guide the operator, through luminous signals, in selecting the instrument indicated by the procedures suggested by the specific operative protocols for the different types of bone or implant. It is also possible to notify the removal of the wrong instrument by means of an acoustic signal.

According to the present invention, the fitting of the implant is guided by measuring the micromotor current, the type of bone is determined using a calibrated tapper which taps the hole previously effected; the measurement must be effected without any pressure on the part of the user, in order not to influence the value of the bone-type detected.

The electronic system measures the current absorbed by the motor for a pre-established time to eliminate the initial measurement errors, and a type of bone corresponds to a certain current measurement (for example 200 ma = bone-type d4, 300 ma = bone-type d3, 400 ma = bone-type d2, 500 ma = bone-type d1); these values are only indicative, the measuring system of the bone-type is in no way influenced by external agents if the measurement is effected without any pressure in the motor (the weight of the motor and type of specific cutter are sufficient). The determination of the bone-type depends on the measurements effected in the laboratory over a period of years, and histological tests; more specifically, the type of bone is determined by the type of instrument (specific reader) and by the electronics which effects the measurement.

Furthermore, by measuring the current absorbed by the motor, it is possible, during the unscrewing phase of the implant, to know if the implant is stable or not, by measuring the average or peak torque (CURRENT MEASURED ON THE MOTOR); the motor must be blocked as soon as there is an indication that the implant is moving from its fitting position, and the torque value reached is indicated both numerically and by graphic visualization. With this monitoring it is possible to verify the stability of the implant.

Furthermore, through the measurement of the current measured on the motor/counterangle and by calculating the integral value for the length of the implant, a numerical value is obtained (INTEGRAL IR measurement) which provides an indication of the stability of the implant and bone compression (DENSITOMETRY).

Furthermore, by inserting in the console the pitch of the implant and depth at which it is to be inserted, the system blocks the motor when the preselected depth is reached, taking into account the number of revs of the motor. The control is effected by monitoring the number of revs of the motor, the pitch of the implant selected, and the established length of the implant. With these three parameters of the electronic system it is possible to know the depth which the implant has reached and it stops at the preset length.

Furthermore, the system also has the possibility of perforating, screwing, tapping, advancing step-by-step by 1 rev degree guaranteeing further precision in the fixing of the implant and clinical evaluations.

The electronic system measures the rate of the motor and the position of the motor. Consequently, by controlling its advancing, it is possible to allow the motor to advance at predefined pitches.

Variations and modifications are possible within the scope of the appended claims.

## Claims

1. A control system of an electric motor for surgical applications comprising a console, an electric micromotor, an intelligent tray and a pedal,
said console comprising an
- electronic power supply section or card (2),
- an electronic motor control section or card (3),
- an electronic logic and display section or card (4) having a keyboard (43), a display (42) and a micro-controller (41) adapted to detect functioning parameters of the micromotor by means of appropriate sensors, and said console being adapted to receive set-ups on the intervention to be effected and being adapted to determine the functioning operative conditions of the motor by comprising means configured to visualize on the display the resistance values encountered by the tool, **characterized in that**
- the sensors are adapted to measure position of the rotation of the motor, the rotation rate and the current absorbed from the motor,
- the console is adapted to determine the type of tool to be used by comprising means configured to visualize on the display the resistance values encountered by the tool,
- the electronic logic and display section or card (4) comprises means adapted to indicate both algebraically and graphically the variations of the resistance encountered by the tool during the operating phases, means adapted to parameterize the variations of the resistance and means adapted to visualize the variations of the resistance in the form of an average value, a peak value and integral value expressed in Newton/cm.

2. The system according to claim 1, in which said intelligent tray is connected to the console and consequently to the logic card in a way configured to verify if the user is using the right tool determined.

3. The system according to claim 1, **characterized in that** the tray comprises a base plate having an electronic/electromechanical sensor adapted to detect the presence of cutters and instruments, and is adapted to indicate the lack thereof by means of an acoustic and/or visual warning signal, and is adapted to supply the micro-controller with this information.

4. The system according to claim 1, **characterized in that** the control card of the motor is adapted to determine the advancing of the motor at predefined pitches by measuring the rate of the motor and the position of the rotation axis of the motor.

5. The system according to claim 1, **characterized in that** it also comprises an electronic hydraulic drive section or card (5) for a peristaltic pump (6) for the cooling liquid.

6. The system according to claim 2, **characterized in that** is comprises means adapted to indicate in each operating phase the type of cutter or tapper to be used in the various sequences of the tray, which in turn is adapted to indicate if the cutter picked up is the right one.

7. The system according to claim 1, **characterized in that** it comprises means adapted to interrupt or send an acoustic warning signal when the desired depth has been reached, based on the pitch of the tapper or implant and the number of revs effected by the motor.

## Patentansprüche

1. Steuersystem eines Elektromotors für chirurgische Anwendungen, umfassend eine Konsole, einen elektrischen Mikromotor, eine intelligente Schale sowie ein Pedal, wobei die Konsole umfasst:
- einen elektronischen Energielieferabschnitt oder eine elektronische Energielieferkarte (2),
- einen elektronischen Motorsteuerabschnitt oder eine elektronische Motorsteuerkarte (3),
- einen elektronischen Logik- und Displayabschnitt oder eine elektronische Logik- und Displaykarte (4) mit einer Tastatur (43), einem Display (42) sowie einem Mikrocontroller (41), der derart angepasst ist, um Funktionsparameter des Mikromotors mittels geeigneter Sensoren zu detektieren, und wobei die Konsole derart angepasst ist, um Einstellungen bezüglich des durchzuführenden Eingriffs aufzunehmen, und derart angepasst ist, um die Funktions-Betriebsbedingungen des Motors **dadurch** zu bestimmen, dass ein Mittel umfasst ist, das derart konfiguriert ist, um die Widerstandswerte, die an dem Werkzeug auftreten, auf dem Display zu visualisieren,
**dadurch gekennzeichnet, dass**
- die Sensoren derart angepasst sind, um die Position der Rotation des Motors, die Drehzahl wie auch den von dem Motor aufgenommenen Strom zu messen,
- die Konsole derart angepasst ist, um den Typ von zu verwendendem Werkzeug **dadurch** zu bestimmen, dass ein Mittel umfasst ist, das derart ausgebildet ist, um die Widerstandswerte, die an dem Werkzeug auftreten, an dem Display zu visualisieren,
- der elektronische Logik- und Displayabschnitt oder die elektronische Logik- und Displaykarte (4) ein Mittel umfasst, das derart angepasst ist, um sowohl algebraisch als auch graphisch die Variationen des Widerstands, der an dem Werkzeug auftritt, während der Betriebsphasen anzugeben, ein Mittel umfasst, das derart angepasst ist, um die Variationen des Widerstands zu parametrisieren, und ein Mittel umfasst, das derart angepasst ist, um die Variationen des Widerstandes in der Form eines Mittelwertes, eines Spitzenwertes und eines Integralwertes, ausgedrückt in Newton/cm, zu visualisieren.

2. System nach Anspruch 1,
wobei die intelligente Schale mit der Konsole und folglich mit der Logikkarte auf eine Weise verbunden ist, die derart ausgebildet ist, um zu verifizieren, ob der Anwender das richtige Werkzeug, das bestimmt wurde, verwendet.

3. System nach Anspruch 1,
**dadurch gekennzeichnet, dass**
die Schale eine Grundplatte mit einem elektronischen/elektromechanischen Sensor umfasst, der derart angepasst ist, um die Anwesenheit von Schneideinrichtungen und Instrumenten zu detektieren, und derart angepasst ist, um deren Mangel mittels eines akustischen und/oder visuellen Warnsignals anzugeben, und derart angepasst ist, um den Mikrocontroller mit dieser Information zu beliefern.

4. System nach Anspruch 1,
**dadurch gekennzeichnet, dass**
die Steuerkarte des Motors derart angepasst ist, um den Vorschub des Motors bei vordefinierten Steigungen bzw. Ganghöhen durch Messung der Rate des Motors und der Position der Rotationsachse des Motors zu bestimmen.

5. System nach Anspruch 1,
**dadurch gekennzeichnet, dass**
es auch einen elektronischen hydraulischen Antriebsabschnitt oder eine elektronische hydraulische Antriebskarte (5) für eine Schlauchpumpe (6) für die Kühlflüssigkeit umfasst.

6. System nach Anspruch 2,
**dadurch gekennzeichnet, dass**
es ein Mittel umfasst, das derart angepasst ist, um in jeder Betriebsphase den Typ von Schneideinrichtung oder Gewindebohreinrichtung anzugeben, der bei den verschiedenen Abfolgen der Schale verwendet werden soll, die seinerseits derart angepasst ist, um anzugeben, ob die Schneideinrichtung, die aufgenommen ist, die richtige ist.

7. System nach Anspruch 1,
**dadurch gekennzeichnet, dass**
es ein Mittel umfasst, das derart angepasst ist, um ein akustisches Warnsignal zu unterbrechen oder zu senden, wenn die gewünschte Tiefe erreicht worden ist, und zwar auf Grundlage der Ganghöhe der Gewindebohreinrichtung oder des Implantats und der Anzahl von durch den Motor bewirkten Umdrehungen.

## Revendications

1. Système de commande d'un moteur électrique pour applications chirurgicales comprenant une console, un micromoteur électrique, un plateau intelligent et une pédale,
ladite console comprenant :
- une section ou carte électronique d'alimentation électrique (2),
- une section ou carte électronique de commande (3) de moteur,
- une section ou carte électronique de logique et d'affichage (4) comportant un clavier (43), un écran (42) et un microcontrôleur (41) adapté pour détecter des paramètres de fonctionnement du micromoteur à l'aide de capteurs appropriés, et ladite console étant adaptée pour recevoir des réglages sur l'intervention à effectuer et étant adaptée pour déterminer les conditions opératives de fonctionnement du moteur en comprenant un moyen configuré pour visualiser sur l'écran les valeurs de résistance rencontrées par l'outil,
**caractérisé en ce que :**
- les capteurs sont adaptés pour mesurer la position de la rotation du moteur, la vitesse de rotation et le courant absorbé par le moteur,
- la console est adaptée pour déterminer le type d'outil à utiliser en comprenant un moyen configuré pour visualiser sur l'écran les valeurs de résistance rencontrées par l'outil,
- la section ou carte électronique de logique et d'affichage (4) comprend un moyen adapté pour indiquer de manière à la fois algébrique et graphique les variations de la résistance rencontrée par l'outil pendant les phases de fonctionnement, un moyen adapté pour paramétrer les variations de la résistance et un moyen adapté pour visualiser les variations de la résistance sous la forme d'une valeur moyenne, d'une valeur maximale et d'une valeur intégrale exprimées en newtons/cm.

2. Système selon la revendication 1, dans lequel ledit plateau intelligent est connecté à la console et en conséquence à la carte logique d'une manière configurée pour vérifier si l'utilisateur emploie le bon outil déterminé.

3. Système selon la revendication 1, **caractérisé en ce que** le plateau comprend une plaque de base comportant un capteur électronique/électromécanique adapté pour détecter la présence d'outils de coupe et d'instruments, et est adapté pour indiquer leur absence au moyen d'un signal d'avertissement acoustique et/ou visuel, et est adapté pour fournir cette information au microcontrôleur.

4. Système selon la revendication 1, **caractérisé en ce que** la carte de commande du moteur est adaptée pour déterminer l'avance du moteur à des pas prédéfinis en mesurant la vitesse du moteur et la position de l'axe de rotation du moteur.

5. Système selon la revendication 1, **caractérisé en ce qu'**il comprend en outre une section ou carte électronique d'entraînement hydraulique (5) pour une pompe péristaltique (6) pour le liquide de refroidissement.

6. Système selon la revendication 2, **caractérisé en ce qu'**il comprend un moyen adapté pour indiquer dans chaque phase de fonctionnement le type d'outil de coupe ou taraud à utiliser dans les diverses séquences du plateau, qui lui-même est adapté pour indiquer si l'outil de coupe choisi est le bon.

7. Système selon la revendication 1, **caractérisé en ce qu'**il comprend un moyen adapté pour interrompre ou envoyer un signal d'avertissement acoustique quand la profondeur voulue a été atteinte, d'après le pas du taraud ou implant et le nombre de révolutions effectuées par le moteur.
